# EUROPEAN PATENT SPECIFICATION

(11) **EP 1 414 409 B1**
(45) Date of publication and mention of the grant of the patent: **14.02.2007**
(21) Application number: 02756952.4
(22) Date of filing: 05.08.2002
(51) Int. Cl.: A61K 9/10, A61K 31/415, A61K 31/635

(54) **STABILIZED ORAL SUSPENSION FORMULATION**
STABILISIERTE ORALE SUSPENSIONSFORMULIERUNG
PREPARATION DE SUSPENSION ORALE STABILISEE

(30) Priority: 06.08.2001 US 310372 P
(43) Date of publication of application: 06.05.2004
(73) Proprietor: Pharmacia Corporation, Chesterfield, MO 63017-1732 (US)
(72) Inventor: LU, Guang, Wei, Ann Arbor, MI 48105 (US)
(74) Representative: Albrecht, Thomas
(86) International application number: PCT/US2002/024746
(87) International publication number: WO 2003/013473

(56) References cited:
- EP-A- 0 257 288
- WO-A-00/32189
- WO-A-01/30342
- WO-A-02/05799
- WO-A-91/08734
- WO-A-93/20798

## Description

### FIELD OF THE INVENTION

The present invention relates to orally deliverable pharmaceutical compositions comprising a drug of low water solubility, and to the use of such compositions in the manufacture of medicaments.

### BACKGROUND OF THE INVENTION

Liquid dosage forms, for example solutions, suspensions, elixirs and syrups, have become an important method by which drugs are delivered to subjects. Such dosage forms are known to provide ease of administration for some patients who have difficulty swallowing solid dosage forms and to increase compliance among certain patient populations by enhancing taste and/or texture of the drug being administered. A yet further advantage of liquid dosage forms is that metering of dosages is continuously variable, providing infinite dose flexibility. The benefits of ease of swallowing and dose flexibility are particularly advantageous for infants, children and the elderly.

In some instances, suspensions can be an advantageous liquid dosage form. For example, some drugs are chemically unstable in solution but are stable when suspended. Additionally, some drugs have a disagreeable taste when in solution but are palatable when administered as undissolved particles.

Unfortunately, many useful drugs are hydrophobic having low solubility in aqueous media and therefore are difficult to formulate as suspensions in aqueous vehicle. Specifically, because of these properties, wetting agents are often required in order to facilitate suspension of hydrophobic drug particles in aqueous media. Surface active wetting agents (*i.e.* surfactants), for example sodium lauryl sulfate, are known to increase suspendability of hydrophobic drugs in aqueous media at least in part by reducing interfacial tension between drug particles and the suspension vehicle, thereby allowing penetration of suspension vehicle into drug aggregates and/or drug particle pores. In addition to advantageous drug-suspending effects, however, use of surfactants in suspensions also often leads to the undesirable result of solubilized and/or dissolved free drug. Since solubilized and/or dissolved drug is susceptible to chemical degradation and/or interaction with other ingredients, suspensions comprising free drug can be chemically unstable.

Another undesirable consequence of using relatively high amounts of surfactant to facilitate suspension of low solubility drugs is that, since surfactants stabilize air bubbles, air entrained during homogenization or shaking of such a suspension tends to remain entrapped therein. As this entrapped air varies depending on agitation force, duration of agitation, and time since agitation, suspension volume necessarily changes making volumetric extraction of uniform doses over time difficult or impossible.

If a drug of low water solubility is to be administered as a suspension, it is desirable that the suspension exhibit slow sedimentation in order to provide suitable dose uniformity. Conversely, where rapid sedimentation occurs, as in the case of an unstructured vehicle, the suspension must be shaken prior to each administration in order to achieve dose uniformity. Other factors being equal (*e.g.* drug particle size, uniformity and density), as viscosity of a particular suspension vehicle increases, velocity of drug particle sedimentation decreases. Therefore, it is desirable that a suspension be suitably viscous so as to inhibit or slow sedimentation of drug particles. However, while such increased viscosity facilitates physical stability, it can also make difficult pouring or administering the suspension. Many suspensions known in the art have failed to adequately address this apparent tradeoff between suitable physical stability and suitable pourability.

U.S. Patent No. 5,112,604 to Beaurline discloses an oral suspension formulation capable of providing dose uniformity for a period of about 90 days. However, no information related to rheology or pourability is disclosed therein.

Deflocculated suspensions are also desirable in many instances. In a structured vehicle, larger particles generally sediment faster than do smaller particles. Therefore, a deflocculated suspension wherein drug particles exist as separate entities (as opposed to loose aggregates or flocs) is desirable with respect to slow sedimentation velocity. A further advantage of deflocculated suspensions is that they do not require addition of flocculating agents. Common flocculating agents such as electrolytes (*e.g.* magnesium aluminum silicate or aluminum chloride) can result in catalyzation of chemical reactions and subsequent degradation of drug or other ingredients. Therefore, with respect to both reduced sedimentation velocity and chemical stability, a deflocculated suspension is more desirable than a flocculated suspension.

Accordingly, there remains a need in the art for suspension formulations of drugs of low water solubility which suspensions are chemically and physically stable and have suitable rheologic properties for pouring and administration.

An illustrative class of drugs for which this need is apparent is the class of selective cyclooxygenase-2 (COX-2) inhibitory drugs of low water solubility. Numerous compounds have been reported having therapeutically and/or prophylactically useful selective cyclooxygenase-2 (COX-2) inhibitory effect, and have been disclosed as having utility in treatment or prevention of specific COX-2 mediated disorders or of such disorders in general. Among such compounds are a large number of substituted pyrazolyl benzenesulfonamides as reported in U.S. Patent No. 5,760,068 to Talley *et al.,* including for example the compound 4-[5-(4-methylphenyl)-3-(trifluoromethyl)-1H-pyrazol-1-yl]benzenesulfonamide, also referred to herein as celecoxib (I), and the compound 4-[5-(3-fluoro-4-methoxyphenyl)-3-difluoromethyl)-1H-pyrazol-1-yl]benzenesulfonamide, also referred to herein as deracoxib (II).

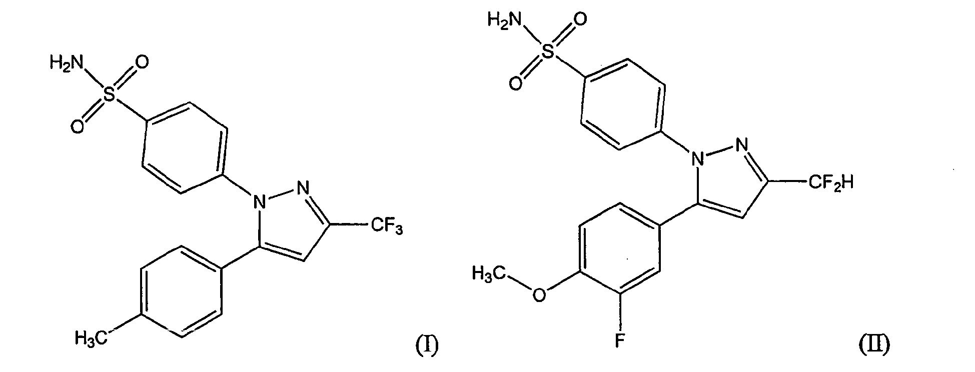

Other compounds reported to have therapeutically and/or prophylactically useful selective COX-2 inhibitory effect are substituted isoxazolyl benzenesulfonamides as reported in U.S. Patent No. 5,633,272 to Talley *et al.,* including the compound 4-[5-methyl-3-phenylisoxazol-4-yl]benzenesulfonamide, also referred to herein as valdecoxib (III).

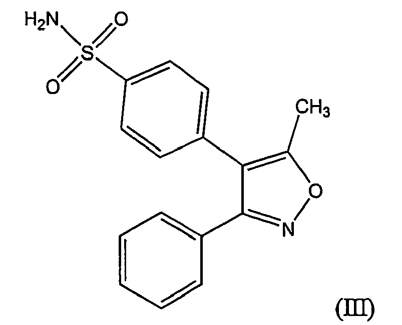

Still other compounds reported to have therapeutically and/or prophylactically useful selective COX-2 inhibitory effect are substituted (methylsulfonyl)phenyl furanones as reported in U.S. Patent No. 5,474,995 to Ducharme *et al.,* including the compound 3-phenyl-4-[4-(methylsulfonyl)phenyl]-5H-furan-2-one, also referred to herein as rofecoxib (IV).

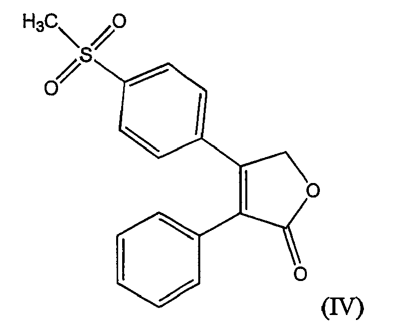

U.S. Patent No. 5,981,576 to Belley *et al.* discloses a further series of (methylsulfonyl)phenyl furanones said to be useful as selective COX-2 inhibitory drugs, including 3-(1-cyclopropylmethoxy)-5,5-dimethyl-4-[4-(methylsulfonyl)phenyl]-5H-furan-2-one and 3-(1-cyclopropylethoxy)-5,5-dimethyl-4-[4-(methylsulfonyl)phenyl]-5H-furan-2-one.

U.S. Patent No. 5,861,419 to Dube *et al.* discloses substituted pyridines said to be useful as selective COX-2 inhibitory drugs, including for example the compound 5-chloro-3-(4-methylsulfonyl)phenyl-2-(2-methyl-5-pyridinyl)pyridine (V), also referred to herein as etoricoxib.

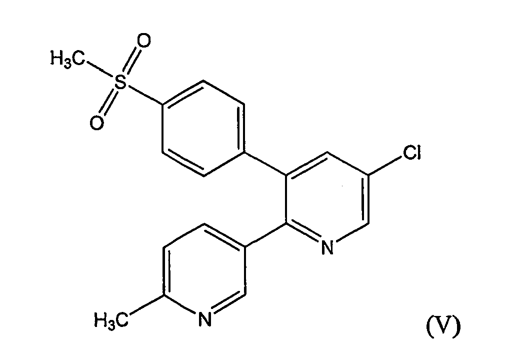

European Patent Application No. 0 863 134 discloses the compound 2-(3,5-difluorophenyl)-3-[4-(methylsulfonyl)phenyl]-2-cyclopenten-1-one said to be useful as a selective COX-2 inhibitory drug.

U.S. Patent No. 6,034,256 discloses a series of benzopyrans said to be useful as selective COX-2 inhibitory drugs, including the compound (S)-6,8-dichloro-2-(trifluoromethyl)-2H-1-benzopyran-3-carboxylic acid (VI).

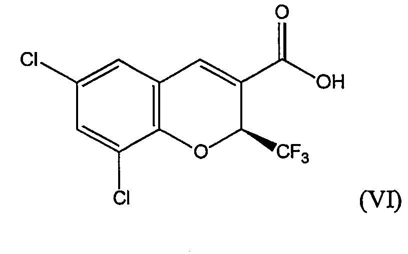

Liquid dosage forms comprising selective COX-2 inhibitory drugs of low water solubility have been disclosed. For example, above-cited U.S. Patent No. 5,760,068 discloses that its subject pyrazolyl benzenesulfonamides, of which celecoxib and deracoxib are examples, can be administered parenterally as isotonic solutions in a range of solvents including polyethylene glycol and propylene glycol.

Above-cited U.S. Patent No. 5,633,272 discloses that its subject isoxazolyl benzenesulfonamides, of which valdecoxib is an example, can be administered parenterally as isotonic solutions in a range of solvents including polyethylene glycol and propylene glycol.

Above-cited U.S. Patent No. 5,474,995 discloses that its subject (methylsulfonyl)phenyl furanones, of which rofecoxib is an example, can be administered parenterally in an isotonic solution in 1,3-butanediol. Also disclosed in U.S. Patent No. 5,474,995 are syrups and elixirs for oral administration, formulated with propylene glycol as a sweetening agent.

Moreover, a suspension of particulate celecoxib in an unstructured vehicle of apple juice was disclosed in co-assigned PCT patent No. WO 00/32189. No information is provided therein on chemical or physical stability of such a composition.

As indicated hereinbelow, treatment with COX-2 inhibitory drugs of low water solubility is indicated in a very wide array of cyclooxygenase-2 mediated disorders and conditions. Therefore, if an orally deliverable, physically and chemically stable, yet still readily pourable, aqueous suspension of a drug of low solubility, for example a selective COX-2 inhibitory drug, could be prepared, a significant advance would be realized in treatment of many conditions and disorders, particularly where easy to swallow dosage forms of various drugs are desired.

### SUMMARY OF THE INVENTION

There is now provided an orally deliverable pharmaceutical composition comprising a drug of low water solubility and an aqueous liquid vehicle that comprises (a) a pharmaceutically acceptable wetting agent, (b) a pharmaceutically acceptable thixotropic thickening agent, and (c) a pharmaceutically acceptable inorganic suspending agent, wherein at least a substantial portion of the drug is suspended in particulate form in the vehicle to form a suspension and wherein the wetting agent, thickening agent, and suspending agent are present in total and relative amounts such that the suspension is thixotropic, substantially deflocculated, and substantially physically stable. More specifically, the invention provides a physically stable, thixotropic, orally deliverable pharmaceutical composition, wherein the composition comprises: a drug having a solubility in water that is no greater than 10 mg/ml at 37°C; and an aqueous liquid vehicle that comprises: (a) a pharmaceutically acceptable wetting agent, (b) a pharmaceutically acceptable thixotropic thickening agent present in a thixotropic amount of from 0.4% to 2% by weight, and (c) a pharmaceutically acceptable inorganic suspending agent; and wherein the thixotropic thickening agent is xanthan gum and wherein the inorganic suspending agent is colloidal silicon dioxide.

In a preferred embodiment, the drug is a selective COX-2 inhibitory drug of low water solubility.

The term "substantially physically stable" as used to describe a suspension herein means that (a) drug particles remain suspended in the suspension vehicle such that dose uniformity is obtainable, as determined for example from two or more aliquots drawn volumetrically, during a stationary room temperature storage period of at least 48 hours after the suspension is prepared, and/or (b) the suspension exhibits substantially uniform drug particle dispersion and substantially no phase separation during a stationary room temperature storage period of at least 48 hours after preparation.

The term "dose uniformity" herein means that, with respect to two or more aliquots drawn volumetrically from the same suspension, either drawn simultaneously or at different time points and drawn from the same or different locations within the suspension, all aliquots contain substantially similar amounts (i.e. ± about 15%) of suspended drug and substantially similar amounts of free drug. An amount of drug in a given volume of suspension can be measured by any suitable method, for example by high performance liquid chromatography.

A "thixotropic" suspension herein is a suspension for which, when an applied stress is continued and/or increased, viscosity substantially decreases. This definition includes suspensions defined elsewhere as being pseudoplastic or shear-thinning.

The term "flocculated" herein refers to drug particles in suspension which coagulate and/or agglomerate together to form loosely packed aggregates or flocs. A "flocculated suspension" herein is a suspension wherein at least a visible portion of all drug particles are flocculated drug particles. The term "substantially deflocculated" herein refers to drug particles in suspension which do not coagulate and/or agglomerate together to form flocs. A "deflocculated suspension" herein is a suspension wherein substantially no visible portion of drug particles are flocculated.

Typically, given a flocculated suspension and a deflocculated suspension having the same drug particle size distribution, drug particle shape, drug particle density, and vehicle viscosity, drug particles in the deflocculated suspension will sediment more slowly than will drug particles in the flocculated suspension. Additionally, when placed in a suspension vehicle of low viscosity, for example an unstructured vehicle, deflocculated drug particles will generally settle to form a tightly packed layer of sediment which is not readily dispersed upon moderate shaking, while flocculated particles will generally settle to form a fluffy, loose layer of sediment which can be redispersed with moderate shaking.

A "structured vehicle" herein is a vehicle comprising one or more viscosity imparting suspending agents which substantially reduce the rate of sedimentation of dispersed particles.

Compositions of the invention are useful for treating and/or preventing a COX-2 mediated disease or disorder.

Compositions of the invention have been found to resolve at least some of the difficulties alluded to above in a surprisingly effective manner. First, compositions of the invention are substantially physically stable. Second, compositions of the invention are thixotropic and therefore are readily poured and administered upon mild agitation. Third, due at least in part to reduced free drug concentration, compositions of the invention have enhanced chemical stability.

Other features of this invention will be in part apparent and in part pointed out hereinafter.

### BRIEF DESCRIPTION OF THE DRAWINGS

Fig. 1 shows sediment volume resulting from centrifugation of Celecoxib Suspensions S1-S3 of Example 1 at 1500 rpm for 5,10 and 30 minutes, as described in Example 3.

Fig. 2 shows sediment volume resulting from centrifugation of Celecoxib Suspensions S1 and S3 of Example 1 at 2500 rpm for 30, 60 and 90 minutes, as described in Example 3.

Fig. 3 shows sediment volume resulting from centrifugation of Celecoxib Suspensions S1-S3 of Example 1 at 3000 rpm for 5, 10 and 20 minutes, as described in Example 3.

### DETAILED DESCRIPTION OF THE INVENTION

Novel pharmaceutical compositions according to the present invention comprise one or more orally deliverable dose units. The term "orally deliverable" herein means suitable for oral administration. The term "oral administration" herein includes any form of delivery of a therapeutic agent or a composition thereof to a subject wherein the agent or composition is placed in the mouth of the subject, whether or not the agent or composition is swallowed. Thus "oral administration" includes buccal and sublingual as well as esophageal administration. Absorption of the agent can occur in any part or parts of the gastrointestinal tract including the mouth, esophagus, stomach, duodenum, jejunum, ileum and colon. The term "dose unit" herein means a portion of a pharmaceutical composition that contains an amount of a therapeutic agent suitable for a single oral administration to provide a therapeutic effect. Typically one dose unit, or a small plurality (up to about 4) of dose units, provides a sufficient amount of the agent to result in the desired effect.

### Drug of low water solubility

Each dose unit or small plurality of dose units comprises, in a therapeutically and/or prophylactically effective total amount, a drug of low water solubility. A "drug of low water solubility" or "poorly water solubility drug" herein refers to any drug or compound having a solubility in water, measured at 37°C, not greater than about 10 mg/ml, and preferably not greater than about 1 mg/ml. It is contemplated that compositions of the invention are especially advantageous for drugs having a solubility in water, measured at 37°C, not greater than about 0.1 mg/ml.

Solubility in water for many drugs can be readily determined from standard pharmaceutical reference books, for example The Merck Index, 11th ed., 1989 (published by Merck & Co., Inc., Rahway, NJ); the United States Pharmacopoeia, 24th ed. (USP 24), 2000; The Extra Pharmacopoeia, 29th ed., 1989 (published by Pharmaceutical Press, London); and the Physicians Desk Reference (PDR), 2001 ed. (published by Medical Economics Co., Montvale, NJ), each of which is individually incorporated herein by reference.

For example, individual drugs of low solubility as defined herein include those drugs categorized as "slightly soluble", "very slightly soluble", "practically insoluble" and "insoluble" in USP 24, pp. 2254-2298; and those drugs categorized as requiring 100 ml or more of water to dissolve 1 g of the drug, as listed in USP 24, pp. 2299-2304.

Illustratively, suitable drugs of low water solubility include, without limitation, drugs from the following classes: abortifacients, ACE inhibitors, α- and β-adrenergic agonists, α- and β-adrenergic blockers, adrenocortical suppressants, adrenocorticotropic hormones, alcohol deterrents, aldose reductase inhibitors, aldosterone antagonists, anabolics, analgesics (including narcotic and non-narcotic analgesics), androgens, angiotensin II receptor antagonists, anorexics, antacids, anthelminthics, antiacne agents, antiallergics, antialopecia agents, antiamebics, antiandrogens, antianginal agents, antiarrhythmics, antiarteriosclerotics, antiarthritic/antirheumatic agents (including selective COX-2 inhibitors), antiasthmatics, antibacterials, antibacterial adjuncts, anticholinergics, anticoagulants, anticonvulsants, antidepressants, antidiabetics, antidiarrheal agents, antidiuretics, antidotes to poison, antidyskinetics, antieczematics, antiemetics, antiestrogens, antifibrotics, antiflatulents, antifungals, antiglaucoma agents, antigonadotropins, antigout agents, antihistaminics, antihyperactives, antihyperlipoproteinemics, antihyperphosphatemics, antihypertensives, antihyperthyroid agents, antihypotensives, antihypothyroid agents, anti-inflammatories, antimalarials, antimanics, antimethemoglobinemics, antimigraine agents, antimuscarinics, antimycobacterials, antineoplastic agents and adjuncts, antineutropenics, antiosteoporotics, antipagetics, antiparkinsonian agents, antipheochromocytoma agents, antipneumocystis agents, antiprostatic hypertrophy agents, antiprotozoals, antipruritics, antipsoriatics, antipsychotics, antipyretics, antirickettsials, antiseborrheics, antiseptics/disinfectants, antispasmodics, antisyphylitics, antithrombocythemics, antithrombotics, antitussives, antiulceratives, antiurolithics, antivenins, antiviral agents, anxiolytics, aromatase inhibitors, astringents, benzodiazepine antagonists, bone resorption inhibitors, bradycardic agents, bradykinin antagonists, bronchodilators, calcium channel blockers, calcium regulators, carbonic anhydrase inhibitors, cardiotonics, CCK antagonists, chelating agents, cholelitholytic agents, choleretics, cholinergics, cholinesterase inhibitors, cholinesterase reactivators, CNS stimulants, contraceptives, debriding agents, decongestants, depigmentors, dermatitis herpetiformis suppressants, digestive aids, diuretics, dopamine receptor agonists, dopamine receptor antagonists, ectoparasiticides, emetics, enkephalinase inhibitors, enzymes, enzyme cofactors, estrogens, expectorants, fibrinogen receptor antagonists, fluoride supplements, gastric and pancreatic secretion stimulants, gastric cytoprotectants, gastric proton pump inhibitors, gastric secretion inhibitors, gastroprokinetics, glucocorticoids, α-glucosidase inhibitors, gonad-stimulating principles, growth hormone inhibitors, growth hormone releasing factors, growth stimulants, hematinics, hematopoietics, hemolytics, hemostatics, heparin antagonists, hepatic enzyme inducers, hepatoprotectants, histamine H₂ receptor antagonists, HIV protease inhibitors, HMG CoA reductase inhibitors, immunomodulators, immunosuppressants, insulin sensitizers, ion exchange resins, keratolytics, lactation stimulating hormones, laxatives/cathartics, leukotriene antagonists, LH-RH agonists, lipotropics, 5-lipoxygenase inhibitors, lupus erythematosus suppressants, matrix metalloproteinase inhibitors, mineralocorticoids, miotics, monoamine oxidase inhibitors, mucolytics, muscle relaxants, mydriatics, narcotic antagonists, neuroprotectives, nootropics, ovarian hormones, oxytocics, pepsin inhibitors, pigmentation agents, plasma volume expanders, potassium channel activators/openers, progestogens, prolactin inhibitors, prostaglandins, protease inhibitors, radio-pharmaceuticals, 5α-reductase inhibitors, respiratory stimulants, reverse transcriptase inhibitors, sedatives/hypnotics, serenics, serotonin noradrenaline reuptake inhibitors, serotonin receptor agonists, serotonin receptor antagonists, serotonin uptake inhibitors, somatostatin analogs, thrombolytics, thromboxane A₂ receptor antagonists, thyroid hormones, thyrotropic hormones, tocolytics, topoisomerase I and II inhibitors, uricosurics, vasomodulators including vasodilators and vasoconstrictors, vasoprotectants, xanthine oxidase inhibitors, and combinations thereof.

Non-limiting illustrative examples of suitable drugs of low water solubility include acetohexamide, acetylsalicylic acid, alclofenac, allopurinol, atropine, benzthiazide, carprofen, celecoxib, chlordiazepoxide, chlorpromazine, clonidine, codeine, codeine phosphate, codeine sulfate, deracoxib, diacerein, diclofenac, diltiazem, estradiol, etodolac, etoposide, etoricoxib, fenbufen, fenclofenac, fenprofen, fentiazac, flurbiprofen, griseofulvin, haloperidol, ibuprofen, indomethacin, indoprofen, ketoprofen, lorazepam, medroxyprogesterone acetate, megestrol, methoxsalen, methylprednisone, morphine, morphine sulfate, naproxen, nicergoline, nifedipine, niflumic, oxaprozin, oxazepam, oxyphenbutazone, paclitaxel, phenindione, phenobarbital, piroxicam, pirprofen, prednisolone, prednisone, procaine, progesterone, pyrimethamine, rofecoxib, sulfadiazine, sulfamerazine, sulfisoxazole, sulindac, suprofen, temazepam, tiaprofenic acid, tilomisole, tolmetic, valdecoxib, etc.

The amount of drug incorporated in a dosage form of the invention can be selected according to known principles of pharmacy. A therapeutically effective amount of drug is specifically contemplated. The term "therapeutically and/or prophylactically effective amount" as used herein refers to an amount of drug that is sufficient to elicit the required or desired therapeutic and/or prophylactic response. Preferably, the therapeutic agent is present in the suspension at a concentration of at least about 0.01 %, at least about 0.1 %, at least about 1 % or at least about 10% up to a concentration of, preferably, no more than about 90%, no more than about 75%, no more than about 50% or no more than about 35% on a weight/weight basis. Unless otherwise indicated, all percentage values given herein are intended to be calculated on a weight/weight basis.

In a particularly preferred embodiment, the drug is a selective COX-2 inhibitory drug of low water solubility. Any such selective COX-2 inhibitory drug known in the art can be used, including without limitation compounds disclosed in the patents and publications listed below.
U.S. Patent No. 5,344,991 to Reitz & Li.
U.S. Patent No. 5,380,738 to Norman *et al.*
U.S. Patent No. 5,393,790 to Reitz *et al.*
U.S. Patent No. 5,401,765 to Lee.
U.S. Patent No. 5,418,254 to Huang & Reitz.
U.S. Patent No. 5,420,343 to Koszyk & Weier.
U.S. Patent No. 5,434,178 to Talley & Rogier.
U.S. Patent No. 5,436,265 to Black *et al*.
Above-cited U.S. Patent No. 5,466,823.
Above-cited U.S. Patent No. 5,474,995.
U.S. Patent No. 5,475,018 to Lee & Bertenshaw.
U.S. Patent No. 5,486,534 to Lee *et al.*
U.S. Patent No. 5,510,368 to Lau *et al.*
U.S. Patent No. 5,521,213 to Prasit *et al.*
U.S. Patent No. 5,536,752 to Ducharme *et al.*
U.S. Patent No. 5,543,297 to Cromlish *et al.*
U.S. Patent No. 5,547,975 to Talley *et al.*
U.S. Patent No. 5,550,142 to Ducharme *et al.*
U.S. Patent No. 5,552,422 to Gauthier *et al.*
U.S. Patent No. 5,585,504 to Desmond *et al.*
U.S. Patent No. 5,593,992 to Adams *et al.*
U.S. Patent No. 5,596,008 to Lee.
U.S. Patent No. 5,604,253 to Lau *et al.*
U.S. Patent No. 5,604,260 to Guay & Li.
U.S. Patent No. 5,616,458 to Lipsky *et al.*
U.S. Patent No. 5,616,601 to Khanna *et al.*
U.S. Patent No. 5,620,999 to Weier *et al.*
Above-cited U.S. Patent No. 5,633,272.
U.S. Patent No. 5,639,780 to Lau *et al.*
U.S. Patent No. 5,643,933 to Talley *et al.*
U.S. Patent No. 5,658,903 to Adams *et al.*
U.S. Patent No. 5,668,161 to Talley *et al.*
U.S. Patent No. 5,670,510 to Huang & Reitz.
U.S. Patent No. 5,677,318 to Lau.
U.S. Patent No. 5,681,842 to Dellaria & Gane.
U.S. Patent No. 5,686,460 to Nicolaï *et al.*
U.S. Patent No. 5,686,470 to Weier *et al.*
U.S. Patent No. 5,696,143 to Talley *et al.*
U.S. Patent No. 5,710,140 to Ducharme *et al.*
U.S. Patent No. 5,716,955 to Adams *et al.*
U.S. Patent No. 5,723,485 to Güngör & Teulon.
U.S. Patent No. 5,739,166 to Reitz *et al.*
U.S. Patent No. 5,741,798 to Lazer *et al.*
U.S. Patent No. 5,756,499 to Adams *et al.*
U.S. Patent No. 5,756,529 to Isakson & Talley.
U.S. Patent No. 5,776,967 to Kreft *et al.*
U.S. Patent No. 5,783,597 to Beers & Wachter.
U.S. Patent No. 5,789,413 to Black *et al.*
U.S. Patent No. 5,807,873 to Nicolaï & Teulon.
U.S. Patent No. 5,817,700 to Dube *et al.*
U.S. Patent No. 5,830,911 to Failli *et al.*
U.S. Patent No. 5,849,943 to Atkinson & Wang.
U.S. Patent No. 5,859,036 to Sartori *et al.*
Above-cited U.S. Patent No. 5,861,419.
U.S. Patent No. 5,866,596 to Sartori & Teulon.
U.S. Patent No. 5,869,524 to Failli.
U.S. Patent No. 5,869,660 to Adams *et al.*
U.S. Patent No. 5,883,267 to Rossen *et al.*
U.S. Patent No. 5,892,053 to Zhi *et al.*
U.S. Patent No. 5,922,742 to Black *et al.*
U.S. Patent No. 5,929,076 to Adams & Garigipati.
U.S. Patent No. 5,932,598 to Talley *et al.*
U.S. Patent No. 5,935,990 to Khanna *et al.*
U.S. Patent No. 5,945,539 to Haruta *et al.*
U.S. Patent No. 5,958,978 to Yamazaki *et al.*
U.S. Patent No. 5,968,958 to Guay *et al.*
U.S. Patent No. 5,972,950 to Nicolaï & Teulon.
U.S. Patent No. 5,973,191 to Marnett & Kalgutkar.
Above-cited U.S. Patent No. 5,981,576.
U.S. Patent No. 5,994,381 to Haruta *et al.*
U.S. Patent No. 6,002,014 to Haruta *et al.*
U.S. Patent No. 6,004,960 to Li *et al.*
U.S. Patent No. 6,005,000 to Hopper *et al.*
U.S. Patent No. 6,020,343 to Belley *et al.*
U.S. Patent No. 6,020,347 to DeLaszlo & Hagmann.
Above-cited U.S. Patent No. 6,034,256.
U.S. Patent No. 6,040,319 to Corley *et al.*
U.S. Patent No. 6,040,450 to Davies *et al.*
U.S. Patent No. 6,046,208 to Adams *et al.*
U.S. Patent No. 6,046,217. to Friesen *et al.*
U.S. Patent No. 6,057,319 to Black *et al.*
U.S. Patent No. 6,063,804 to De Nanteuil *et al.*
U.S. Patent No. 6,063,807 to Chabrier de Lassauniere & Broquet.
U.S. Patent No. 6,071,954 to LeBlanc *et al.*
U.S. Patent No. 6,077,868 to Cook *et al.*
U.S. Patent No. 6,077,869 to Sui & Wachter.
U.S. Patent No. 6,083,969 to Ferro *et al.*
U.S. Patent No. 6,096,753 to Spohr *et al.*
U.S. Patent No. 6,133,292 to Wang *et al.*
International Patent Publication No. WO 94/15932.
International Patent Publication No. WO 96/19469.
International Patent Publication No. WO 96/26921.
International Patent Publication No. WO 96/31509.
International Patent Publication No. WO 96/36623.
International Patent Publication No. WO 96/38418.
International Patent Publication No. WO 97/03953.
International Patent Publication No. WO 97/10840.
International Patent Publication No. WO 97/13755.
International Patent Publication No. WO 97/13767.
International Patent Publication No. WO 97/25048.
International Patent Publication No. WO 97/30030.
International Patent Publication No. WO 97/34882.
International Patent Publication No. WO 97/46524.
International Patent Publication No. WO 98/04527.
International Patent Publication No. WO 98/06708.
International Patent Publication No. WO 98/07425.
International Patent Publication No. WO 98/17292.
International Patent Publication No. WO 98/21195.
International Patent Publication No. WO 98/22457.
International Patent Publication No. WO 98/32732.
International Patent Publication No. WO 98/41516.
International Patent Publication No. WO 98/43966.
International Patent Publication No. WO 98/45294.
International Patent Publication No. WO 98/47871.
International Patent Publication No. WO 99/01130.
International Patent Publication No. WO 99/01131.
International Patent Publication No. WO 99/01452.
International Patent Publication No. WO 99/01455.
International Patent Publication No. WO 99/10331.
International Patent Publication No. WO 99/10332.
International Patent Publication No. WO 99/11605.
International Patent Publication No. WO 99/12930.
International Patent Publication No. WO 99/14195.
International Patent Publication No. WO 99/14205.
International Patent Publication No. WO 99/15505.
International Patent Publication No. WO 99/23087.
International Patent Publication No. WO 99/24404.
International Patent Publication No. WO 99/25695.
International Patent Publication No. WO 99/35130.
International Patent Publication No. WO 99/61016.
International Patent Publication No. WO 99/61436.
International Patent Publication No. WO 99/62884.
International Patent Publication No. WO 99/64415.
International Patent Publication No. WO 00/01380.
International Patent Publication No. WO 00/08024.
International Patent Publication No. WO 00/10993.
International Patent Publication No. WO 00/13684.
International Patent Publication No. WO 00/18741.
International Patent Publication No. WO 00/18753.
International Patent Publication No. WO 00/23426.
Above-cited International Patent Publication No. WO 00/24719.
International Patent Publication No. WO 00/26216.
International Patent Publication No. WO 00/31072.
International Patent Publication No. WO 00/40087.
International Patent Publication No. WO 00/56348.
European Patent Application No. 0 799 823.
European Patent Application No. 0 846 689.
Above-cited European Patent Application No. 0 863 134.
European Patent Application No. 0 985 666.

Compositions of the invention are especially useful for compounds having the formula (VII):

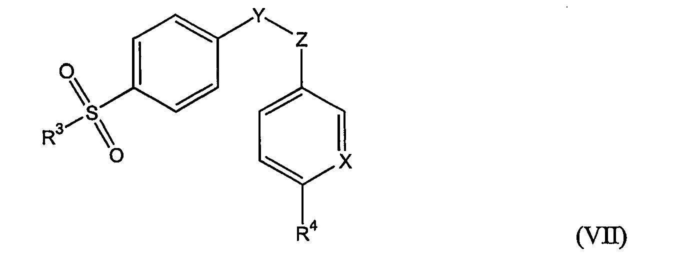

where R³ is a methyl or amino group, R⁴ is hydrogen or a C₁₋₄ alkyl or alkoxy group, X is N or CR⁵ where R⁵ is hydrogen or halogen, and Y and Z are independently carbon or nitrogen atoms defining adjacent atoms of a five- to six-membered ring that is unsubstituted or substituted at one or more positions with oxo, halo, methyl or halomethyl groups. Preferred such five- to six-membered rings are cyclopentenone, furanone, methylpyrazole, isoxazole and pyridine rings substituted at no more than one position.

Illustratively, celecoxib, deracoxib, valdecoxib, rofecoxib, etoricoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid and 2-(3,4-difluorophenyl)-4-(3-hydroxy-3-methyl-1-butoxy)-5-[4-(methylsulfonyl)phenyl]-3-(2H)-pyridazinone, more particularly celecoxib, valdecoxib, rofecoxib and etoricoxib, and still more particularly celecoxib and valdecoxib, are useful in compositions of the invention.

The invention is illustrated herein with particular reference to celecoxib, and it will be understood that any other drug of low solubility in water can, if desired, be substituted in whole or in part for celecoxib in compositions herein described.

Where the drug is celecoxib, the composition typically comprises celecoxib in a therapeutically and/or prophylactically effective total amount of about 2 mg to about 1000 mg per dose unit. Where the drug is a selective COX-2 inhibitory drug other than celecoxib, the amount of the drug per dose unit is therapeutically equivalent to about 2 mg to about 1000 mg of celecoxib.

It will be understood that a therapeutically and/or prophylactically effective amount of a drug for a subject is dependent *inter alia* on the body weight of the subject. A "subject" herein to which a therapeutic agent or composition thereof can be administered includes a human subject or patient of either sex and of any age, and also includes any nonhuman animal, particularly a domestic or companion animal, illustratively a cat, dog or horse.

Where the subject is an infant, child or a small animal (*e.g*., a dog), for example, an amount of celecoxib relatively low in the preferred range of about 2 mg to about 1000 mg is likely to be consistent with therapeutic effectiveness. Where the subject is an adult human or a large animal (*e.g.*, a horse), therapeutic effectiveness is likely to require dose units containing a relatively greater amount of celecoxib. For an adult human, a therapeutically effective amount of celecoxib per dose unit in a composition of the present invention is typically about 50 mg to about 400 mg. Especially preferred amounts of celecoxib per dose unit are about 100 mg to about 200 mg.

For other selective COX-2 inhibitory drugs, an amount of the drug per dose unit can be in a range known to be therapeutically effective for such drugs. Preferably, the amount per dose unit is in a range providing therapeutic equivalence to celecoxib in the dose ranges indicated immediately above.

A celecoxib suspension composition of the present invention preferably comprises about 0.01 to about 15%, more preferably about 0.01 to about 10%, and yet more preferably about 0.01 to about 5% by total weight, of celecoxib. Generally, these concentrations will correspond to celecoxib in an amount of about 1% to about 90%, more preferably about 1% to about 75%, more preferably about 1% to about 50%, and still more preferably about 1% to about 35%, by weight of all dry, non-liquid components.

Where the selective COX-2 inhibitory drug is other than celecoxib and is therapeutically effective at lower dosages than celecoxib, the minimum amount of COX-2 inhibitory drug in suspension can be lower than that indicated immediately above for celecoxib; for example, in the case of valdecoxib, the drug can be present in an amount of about 0.1% by total weight.

Preferably about 50% to 100%, more preferably about 75% to 100%, still more preferably about 85% to 100%, and yet more preferably substantially all of the celecoxib present in compositions of the invention is suspended in particulate form. It is also preferred in compositions of the invention that less than about 25%, more preferably less than about 10%, yet more preferably less than about 5%, and still more preferably substantially no portion, of the celecoxib is in dissolved and/or solubilized form.

Particulate celecoxib present in suspension compositions of the invention preferably has a D₉₀ particle size of about 0.5 µm to about 50 µm, more preferably about 0.5 µm to about 40 µm, and still more preferably 0.5 µm to about 30 µm. D₉₀ is defined herein as a linear measure of diameter having a value such that 90% by weight of particles in the formulation, in the longest dimension of the particles, are smaller than that diameter.

### Wetting agent

One or more wetting agents are present in suspension compositions of the invention. The one or more wetting agent is believed to facilitate suspension of drug particles of low water solubility, but may have other functions.

Surfactants, hydrophilic polymers and certain clays can be useful as wetting agents to aid in suspension of a hydrophobic drug such as celecoxib. Surfactants, including nonionic, anionic, cationic and zWitterionic surfactants, are preferred wetting agents in suspension compositions of the invention. Non-limiting examples of surfactants that can be used as wetting agents in compositions of the invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e.g.*, Labrasol^{™} of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e.g.*, Tween^{™} 80 of ICI), propylene glycol fatty acid esters, for example propylene glycol laurate (*e.g.*, Lauroglycol^{™} of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. A preferred class of wetting agents is polyoxyethylene sorbitan esters.

Benzalkonium chloride, benzethonium chloride, cetylpyridinium chloride, dioctyl sodium sulfosuccinate, nonoxynol 9, nonoxynol 10, octoxynol 9, polyoxyethylene (8) caprylic/capric mono- and diglycerides, polyoxyethylene (35) castor oil, polyoxyethylene (20) cetostearyl ether, polyoxyethylene (40) hydrogenated castor oil, polyoxyethylene (10) oleyl ether, polyoxyethylene (40) stearate, polysorbate 20, polysorbate 40, polysorbate 60, polysorbate 80, propylene glycol laurate, sodium lauryl sulfate, sorbitan monolaurate, sorbitan monooleate, sorbitan monopalmitate, sorbitan monostearate and tyloxapol are preferred wetting agents. Polysorbate 80 is a particularly preferred wetting agent.

Too much or inappropriate selection of wetting agent, for example surfactants, can be undesirable for several reasons. High free surfactant concentration can result in flocculated drug particles which sediment more rapidly than do deflocculated drug particles. High free surfactant concentration can also lead to dissolved and/or solubilized drug which, by comparison with the same drug in particulate form, can be more susceptible to chemical degradation and can produce an unpleasant taste. Therefore, in a particularly preferred embodiment, one or more wetting agents are present in a total amount sufficient to facilitate drug particle suspension, but in a substantially non-drug-solubilizing and non-drug-dissolving amount.

In another preferred embodiment, one or more wetting agents are present in total and relative amounts sufficient to suspend in particulate form at least about 75%, more preferably at least about 85%, and still more preferably substantially all of the drug, but in total and relative amounts less than will result in about 25% or more, preferably in about 10% or more, and more preferably in substantially any portion, of solubilized and/or dissolved drug being present in the composition.

Too much surfactant can also lead to retention of entrapped air within a suspension composition of the invention. Generally, when a suspension is homogenized during preparation, air is entrained therein. Without being bound by theory, surfactants and other wetting agents are believed to stabilize air bubbles at least in part by decreasing surface tension at the bubble surface. Thus, by comparison with a suspension comprising relatively high amounts of free surfactant, a suspension having decreased free surfactant concentration is believed to exhibit less stable entrained air bubbles and, subsequently, to exhibit improved dose/volume uniformity.

Generally, one or more wetting agent(s) constitute, in total, about 0.01 % to about 2%, preferably about 0.1% to about 1.5%, and more preferably about 0.25% to about 0.75%, of the total weight of the composition.

### Thixotropic thickening agent

Compositions of the invention comprise one or more thixotropic thickening agents. Thixotropic thickening agents are natural or synthetic polymers which, when added to aqueous medium, impart thixotropic characteristics thereto. The thixotropic thickening agent is xanthan gum. Other thixotropic thickening agents are cellulosic polymers such as methylcellulose, microcrystalline cellulose (*e.g. Avicel* of FMC Corp.), polyvinylpyrrolidone, hydroxypropyl methycellulose, etc. and polysaccharide gums, such as guar, acacia, and tragacanth gums.

The thixotropic thickening agent is present in compositions of the invention in a total amount of 0.4% to 2%, on a weight/weight basis. While the term polysaccharide gum is sometimes used herein to refer to a thixotropic thickening agent, such agents are not limited to polysaccharide gums.

### Inorganic suspending agent

The inorganic suspending agent used in compositions of the invention is colloidal silicon dioxide.

While colloidal silicon dioxide at relatively high concentrations (*e.g* about 5 to about 15%) is known to impart thixotropic characteristics in certain gel and semi-solid preparations, we have unexpectedly discovered that a small amount of colloidal silicon dioxide (*e.g.* about 2% or less) has a synergistic effect with polysaccharide gum, enhancing thixotropic characteristics of compositions of the invention. Specifically, the presence of both colloidal silicon dioxide and a polysaccharide gum in compositions of the invention enhances thixotropic characteristics more than was expected from addition of the individual thixotropy-enhancing effects of the two respective components.

Surprisingly, we have also discovered that addition of a small amount of colloidal silicon dioxide to suspension compositions of the invention leads to improved chemical stability of drug present therein. Without being held to a particular theory, it is presently believed that colloidal silicon dioxide in compositions of the invention adsorbs wetting agent resulting in lower free wetting agent concentration compared to an otherwise similar composition having no colloidal silicon dioxide; in turn, the lower free wetting agent concentration is believed to result in reduced free drug concentration and subsequently, an increased proportion of drug present in particulate form which is less susceptible to chemical degradation than is free drug.

Colloidal silicon dioxide in compositions of the invention also provides the surprising advantage of reduced retention of entrapped air and subsequently, improved dose uniformity. As is described above, free wetting agent can stabilize air bubbles entrapped within a suspension. Without being bound by theory, adsorption of free wetting agent by colloidal silicon dioxide present in compositions of the invention is believed to result in reduced free wetting agent concentration and, subsequently, more unstable entrapped air bubbles.

Any pharmaceutically acceptable source of colloidal silicon dioxide can be used in compositions of the invention. Non-limiting synonyms for, and examples of, suitable colloidal silicon dioxides include *Aerosil*^{™} of Degussa, *Cab-O-Sil*^{™} of Cabot Corp., fumed silica, light anhydrous silicic acid, silicic anhydride, silicon dioxide fumed, colloidal silica, *etc.*

In a presently preferred embodiment, the colloidal silicon dioxide has a specific surface area of about 50 to about 400, more preferably about 100 to about 400, and still more preferably about 150 to about 400 m²/g. Preferred colloidal silicon dioxides also have a weight average particle diameter of about 7 to about 40 nm, and more preferably about 10 to about 25 nm. *Cab-O-Sil*^{™} is a particularly preferred colloidal silicon dioxide for use in compositions of the invention.

One or more inorganic suspending agents are generally present in compositions of the invention in a total amount of about 0.01% to about 3.0%, preferably about 0.1% to about 2.0%, and more preferably about 0.25% to about 1.0%, by weight.

### Thixotropic features

A beneficial feature of compositions of the invention is that at steady state they are substantially physically stable yet upon mild agitation they are readily pourable. The term "readily pourable" herein means that the composition will be easily poured from a suitable container such as a jar or bottle. The relatively high yield value of compositions of the invention promotes physical stability. "Yield value" herein (also referred to herein as maximum viscosity) is a measure of a suspension's initial resistance to flow under stress.

Total and relative amounts of wetting agent, inorganic suspending agent, and thixotropic thickening agent can be readily optimized within the ranges herein provided by one skilled in the art in order to provide suitable rheologic characteristics, deflocculation of drug particles, and minimal free drug concentration. Such optimization will take into account other relevant factors such as the particular drug, drug particle size, uniformity and density, drug concentration, additional excipients present, desired shelf life, *etc.*

For practical purposes, whether a particular composition is thixotropic herein can be determined according to Test I.

### Test I:

After standing at rest for at least 60 minutes, a 1 ml sample is drawn from a test suspension and is placed into a P45 Cup of a HAAKE CV100 viscometer (equipped with a PK30-4 spindle) which is maintained at 25 °C throughout the test.

A sheer stress is applied to the sample and is incrementally increased from 0 to 3 sec⁻¹ over a period of 3 minutes and then incrementally decreased from 3 to 0 sec⁻¹ over another period of 3 minutes. Dynamic viscosity is measured at a sheer rate of 2 sec⁻¹ where a steady state is obtained. Yield value is calculated using linear regression of the data from 2 sec⁻¹ to 3 sec⁻¹.

If, during the test, the sample has a maximum viscosity of about 5 to about 50 Pa•s and a dynamic viscosity, measured at 2 sec⁻¹, of about 0.3 to about 20 Pa•s, the composition is deemed thixotropic.

Preferably, the thixotropic thickening agent and inorganic suspending agent are present in total and relative amounts such that, when a composition of the invention is analyzed according to Test I, the composition has a maximum viscosity of about 5 to about 40, more preferably about 5 to about 30, and still more preferably about 5 to about 25 Pa•s and, at a shear rate of 2 sec⁻¹, the composition has a dynamic viscosity of about 0.3 to about 20, preferably about 0.4 to about 15, and yet more preferably about 0.5 to about 7 Pa•s.

Generally, the thixotropic thickening agent and inorganic suspending agent will be present in a weight ratio of about 10:1 to about 1:10, preferably about 5:1 to about 1:5, and more preferably about 2:1 to about 1:2.

Importantly, because compositions of the invention are substantially deflocculated and substantially physically stable, shaking or agitation is not required in order to re-disperse drug particles or to mix the suspension prior to administration. Moderate shaking will facilitate pourability of suspension compositions of the invention.

### Pharmaceutical excipients

Compositions of the invention can comprise any additional pharmaceutically acceptable excipients. The term "excipient" herein means any substance, not itself a therapeutic agent, used as a carrier or vehicle for delivery of a therapeutic agent to a subj ect or added to a pharmaceutical composition to improve its handling, storage, disintegration, dispersion, dissolution, release or organoleptic properties or to permit or facilitate formation of a dose unit of the composition into a discrete article such as a capsule suitable for oral administration. Excipients can include, by way of illustration and not limitation, diluents, polymers, substances added to mask or counteract a disagreeable taste or odor, flavors, dyes, preservatives, fragrances, and substances added to improve appearance of the composition.

Compositions of the invention can be prepared by any suitable process, not limited to processes described herein. One illustrative process comprises admixing celecoxib, one or more wetting agents, a polysaccharide gum, and silicon dioxide together with any other desired excipients to form a powder blend, and homogenizing the powder blend together with water for form a suspension.

### Utility of compositions of the invention

Where compositions of the invention comprise a COX-2 inhibitory drug of low water solubility, they are useful in treatment and prevention of a very wide range of disorders mediated by COX-2, including but not restricted to disorders characterized by inflammation, pain and/or fever. Such compositions are especially useful as anti-inflammatory agents, such as in treatment of arthritis, with the additional benefit of having significantly less harmful side effects than compositions of conventional nonsteroidal anti-inflammatory drugs (NSAIDs) that lack selectivity for COX-2 over COX-1. In particular, compositions of the invention have reduced potential for gastrointestinal toxicity and gastrointestinal irritation including upper gastrointestinal ulceration and bleeding, reduced potential for renal side effects such as reduction in renal function leading to fluid retention and exacerbation of hypertension, reduced effect on bleeding times including inhibition of platelet function, and possibly a lessened ability to induce asthma attacks in aspirin-sensitive asthmatic subjects, by comparison with compositions of conventional NSAIDs. Thus compositions of the invention are particularly useful as an alternative to conventional NSAIDs where such NSAIDs are contraindicated, for example in patients with peptic ulcers, gastritis, regional enteritis, ulcerative colitis, diverticulitis or with a recurrent history of gastrointestinal lesions; gastrointestinal bleeding, coagulation disorders including anemia such as hypoprothrombinemia, hemophilia or other bleeding problems; kidney disease; or in patients prior to surgery or patients taking anticoagulants.

Contemplated compositions are useful to treat a variety of arthritic disorders, including but not limited to rheumatoid arthritis, spondyloarthropathies, gouty arthritis, osteoarthritis, systemic lupus erythematosus and juvenile arthritis.

Such compositions are useful in treatment of asthma, bronchitis, menstrual cramps, preterm labor, tendinitis, bursitis, allergic neuritis, cytomegalovirus infectivity, apoptosis including HIV-induced apoptosis, lumbago, liver disease including hepatitis, skin-related conditions such as psoriasis, eczema, acne, burns, dermatitis and ultraviolet radiation damage including sunburn, and post-operative inflammation including that following ophthalmic surgery such as cataract surgery or refractive surgery.

Such compositions are useful to treat gastrointestinal conditions such as inflammatory bowel disease, Crohn's disease, gastritis, irritable bowel syndrome and ulcerative colitis.

Such compositions are useful in treating inflammation in such diseases as migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, and the like.

Such compositions are useful in treatment of ophthalmic diseases, such as retinitis, conjunctivitis, retinopathies, uveitis, ocular photophobia, and of acute injury to the eye tissue.

Such compositions are useful in treatment of pulmonary inflammation, such as that associated with viral infections and cystic fibrosis, and in bone resorption such as that associated with osteoporosis.

Such compositions are useful for treatment of certain central nervous system disorders, such as cortical dementias including Alzheimer's disease, neurodegeneration, and central nervous system damage resulting from stroke, ischemia and trauma. The term "treatment" in the present context includes partial or total inhibition of dementias, including Alzheimer's disease, vascular dementia, multi-infarct dementia, pre-senile dementia, alcoholic dementia and senile dementia.

Such compositions are useful in treatment of allergic rhinitis, respiratory distress syndrome, endotoxin shock syndrome and liver disease.

Such compositions are useful in treatment of pain, including but not limited to postoperative pain, dental pain, muscular pain, and pain resulting from cancer. For example, such compositions are useful for relief of pain, fever and inflammation in a variety of conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, burns, and trauma following surgical and dental procedures.

Such compositions are useful for treating and preventing inflammation-related cardiovascular disorders, including vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries.

Such compositions are useful in treatment of angiogenesis-related disorders in a subject, for example to inhibit tumor angiogenesis. Such compositions are useful in treatment of neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and neovascular glaucoma; ulcerative diseases such as gastric ulcer; pathological, but non-malignant, conditions such as hemangiomas, including infantile hemaginomas, angiofibroma of the nasopharynx and avascular necrosis of bone; and disorders of the female reproductive system such as endometriosis.

Such compositions are useful in prevention and treatment of benign and malignant tumors and neoplasia including cancer, such as colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer, skin cancer such as squamous cell and basal cell cancers, prostate cancer, renal cell carcinoma, and other known cancers that effect epithelial cells throughout the body. Neoplasias for which compositions of the invention are contemplated to be particularly useful are gastrointestinal cancer, Barrett's esophagus, liver cancer, bladder cancer, pancreatic cancer, ovarian cancer, prostate cancer, cervical cancer, lung cancer, breast cancer and skin cancer. Such compositions can also be used to treat fibrosis that occurs with radiation therapy. Such compositions can be used to treat subjects having adenomatous polyps, including those with familial adenomatous polyposis (FAP). Additionally, such compositions can be used to prevent polyps from forming in patients at risk of FAP.

Such compositions inhibit prostanoid-induced smooth muscle contraction by inhibiting synthesis of contractile prostanoids and hence can be of use in treatment of dysmenorrhea, premature labor, asthma and eosinophil-related disorders. They also can be of use for decreasing bone loss particularly in postmenopausal women (*i.e.*, treatment of osteoporosis), and for treatment of glaucoma.

Preferred uses for compositions of the invention are for treatment of rheumatoid arthritis and osteoarthritis, for pain management generally (particularly post-oral surgery pain, post-general surgery pain, post-orthopedic surgery pain, and acute flares of osteoarthritis), for treatment of Alzheimer's disease, and for colon cancer chemoprevention.

For treatment of rheumatoid arthritis or osteoarthritis, compositions of the invention can be used to provide a daily dosage of celecoxib of about 50 mg to about 1000 mg, preferably about 100 mg to about 600 mg, more preferably about 150 mg to about 500 mg, still more preferably about 175 mg to about 400 mg, for example about 200 mg. A daily dose of celecoxib of about 0.7 to about 13 mg/kg body weight, preferably about 1.3 to about 8 mg/kg body weight, more preferably about 2 to about 6.7 mg/kg body weight, and still more preferably about 2.3 to about 5.3 mg/kg body weight, for example about 2.7 mg/kg body weight, is generally appropriate when administered in a composition of the invention. The daily dose can be administered in one to about four doses per day, preferably one or two doses per day.

For treatment of Alzheimer's disease or cancer, compositions of the invention can be used to provide a daily dosage of celecoxib of about 50 mg to about 1000 mg, preferably about 100 mg to about 800 mg, more preferably about 150 mg to about 600 mg, and still more preferably about 175 mg to about 400 mg, for example about 400 mg. A daily dose of about 0.7 to about 13 mg/kg body weight, preferably about 1.3 to about 10.7 mg/kg body weight, more preferably about 2 to about 8 mg/kg body weight, and still more preferably about 2.3 to about 5.3 mg/kg body weight, for example about 5.3 mg/kg body weight, is generally appropriate when administered in a composition of the invention. The daily dose can be administered in one to about four doses per day, preferably one or two doses per day.

For pain management, compositions of the invention can be used to provide a daily dosage of celecoxib of about 50 mg to about 1000 mg, preferably about 100 mg to about 600 mg, more preferably about 150 mg to about 500 mg, and still more preferably about 175 mg to about 400 mg, for example about 200 mg. A daily dose of celecoxib of about 0.7 to about 13 mg/kg body weight, preferably about 1.3 to about 8 mg/kg body weight, more preferably about 2 to about 6.7 mg/kg body weight, and still more preferably about 2.3 to about 5.3 mg/kg body weight, for example about 2.7 mg/kg body weight, is generally appropriate when administered in a composition of the invention. The daily dose can be administered in one to about four doses per day. Administration at a rate of one 50 mg dose unit four times a day, one 100 mg dose unit or two 50 mg dose units twice a day or one 200 mg dose unit, two 100 mg dose units or four 50 mg dose units once a day is preferred.

For selective COX-2 inhibitory drugs other than celecoxib, appropriate doses can be selected by reference to the patent literature cited hereinabove.

Besides being useful for human treatment, compositions of the invention are useful for veterinary treatment of companion animals, exotic animals, farm animals, and the like, particularly mammals. More particularly, compositions of the invention are useful for treatment of COX-2 mediated disorders in horses, dogs and cats.

The compositions of the present invention can be used in a therapeutic method of treating a condition or disorder where treatment with a COX-2 inhibitory drug is indicated, the method comprising oral administration of a composition of the invention to a subject in need thereof. The dosage regimen to prevent, give relief from, or ameliorate the condition or disorder preferably corresponds to once-a-day or twice-a-day treatment, but can be modified in accordance with a variety of factors. These include the type, age, weight, sex, diet and medical condition of the subject and the nature and severity of the disorder. Thus, the dosage regimen actually employed can vary widely and can therefore deviate from the preferred dosage regimens set forth above.

Initial treatment can begin with a dose regimen as indicated above. Treatment is generally continued as necessary over a period of several weeks to several months or years until the condition or disorder has been controlled or eliminated. Subjects undergoing treatment with a composition of the invention can be routinely monitored by any of the methods well known in the art to determine effectiveness of therapy. Continuous analysis of data from such monitoring permits modification of the treatment regimen during therapy so that optimally effective doses are administered at any point in time, and so that the duration of treatment can be determined. In this way, the treatment regimen and dosing schedule can be rationally modified over the course of therapy so that the lowest amount of the composition exhibiting satisfactory effectiveness is administered, and so that administration is continued only for so long as is necessary to successfully treat the condition or disorder.

The present compositions can be used in combination therapies with opioids and other analgesics, including narcotic analgesics, Mu receptor antagonists, Kappa receptor antagonists, non-narcotic (i.e. non-addictive) analgesics, monoamine uptake inhibitors, adenosine regulating agents, cannabinoid derivatives, Substance P antagonists, neurokinin-1 receptor antagonists and sodium channel blockers, among others. Preferred combination therapies comprise use of a composition of the invention with one or more compounds selected from the group consisting of aceclofenac, acemetacin, e-acetamidocaproic acid, acetaminophen, acetaminosalol, acetanilide, acetylsalicylic acid (aspirin), S-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, *p*-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil, lonazolac, lornoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen and zomepirac (see The Merck Index, 12th Edition (1996), Therapeutic Category and Biological Activity Index, lists therein headed "Analgesic", "Anti-inflammatory" and "Antipyretic").

Particularly preferred combination therapies comprise use of a composition of the invention with an opioid compound, more particularly where the opioid compound is codeine, meperidine, morphine or a derivative thereof.

The compound to be administered in combination with a selective COX-2 inhibitory drug can be formulated separately from the drug or co-formulated with the drug in a composition of the invention. Where a selective COX-2 inhibitory drug is co-formulated with a second drug, for example an opioid drug, the second drug can be formulated in immediate-release, rapid-onset, sustained-release or dual-release form.

### EXAMPLES

The following Examples are provided for illustrative purposes. The Examples will permit better understanding of the invention and better perception of its advantages.

### Example 1

Six celecoxib suspensions were prepared comprising components shown in Table 1. All components were admixed, and suspensions were homogenized for 5 minutes using a Silverson Laboratory Mixer Emulsifier.

**Table 1. Composition (%) of Celecoxib Suspensions S1-S6.**

| Component | **S1*** | **S2*** | S3 | S4 | S5 | **S6*** |
|---|---|---|---|---|---|---|
| Celecoxib | 2 | 2 | 2 | 2 | 2 | 2 |
| Xanthan gum | 0.5 | - | 0.5 | 0.5 | 0.5 | - |
| Colloidal silica | - | 0.5 | 0.5 | 1.0 | 2.0 | 1.0 |
| Polysorbate 80 | 0.5 | 0.5 | 0.5 | 0.5 | 0.5 | 0.5 |
| Sucrose | 30 | 30 | 30 | 30 | 30 | 30 |
| Citric acid | 1.31 | 1.31 | 1.31 | 1.31 | 1.31 | 1.31 |
| Sodium citrate | 1.11 | 1.11 | 1.11 | 1.11 | 1.11 | 1.11 |
| Tutti fruti | 0.1 | 0.1 | 0.1 | 0.1 | 0.1 | 0.1 |
| Sodium benzoate | 0.2 | 0.2 | 0.2 | 0.2 | 0.2 | 0.2 |
| FD&C red #40 | 0.1 | 0.1 | 0.1 | 0.1 | 0.1 | 0.1 |
| Water | to 100 | to 100 | to 100 | to 100 | to 100 | to 100 |

| | | | | | | |
|---|---|---|---|---|---|---|
| * For comparison. | | | | | | |

### Example 2

Celecoxib Suspensions S1-S6 of Example 1 were maintained at room temperature for 24 hours before sampling. A 1.5 ml sample of each suspension was then drawn and individually centrifuged at 15,800 g for 60 minutes or at 43,000 g for 10 minutes. After centrifugation, 0.1 to 0.5 ml aliquots of supernatant were removed from each suspension with a pipette; each aliquot was individually diluted with methanol (from 1:2 to 2:10) to precipitate xanthan gum. The diluted samples were then centrifuged at 15,800 g for 15 minutes. Free drug concentration, shown in Table 2, was determined for Suspensions S1 and S3-S5 via high performance liquid chromatography (HPLC) analysis of the diluted supernatant.

**Table 2. Free drug concentration (mg/ml) in Celecoxib Suspensions S1 and S3-S5.**

| | |
|---|---|
| **Celecoxib Suspension** | **Free drug concentration** |
| S1 | 0.311 |
| S3 | 0.187 |
| S4 | 0.009 |
| S5 | 0.010 |

As shown in Table 2, Celecoxib Suspensions comprising colloidal silicon dioxide (S3-S5) had reduced free drug concentration compared to Celecoxib Suspension S1 which comprised no colloidal silicon dioxide. These data suggest that drug in Celecoxib Suspensions comprising colloidal silicon dioxide are less susceptible to chemical degradation than in those suspensions without colloidal silicon dioxide.

### Example 3

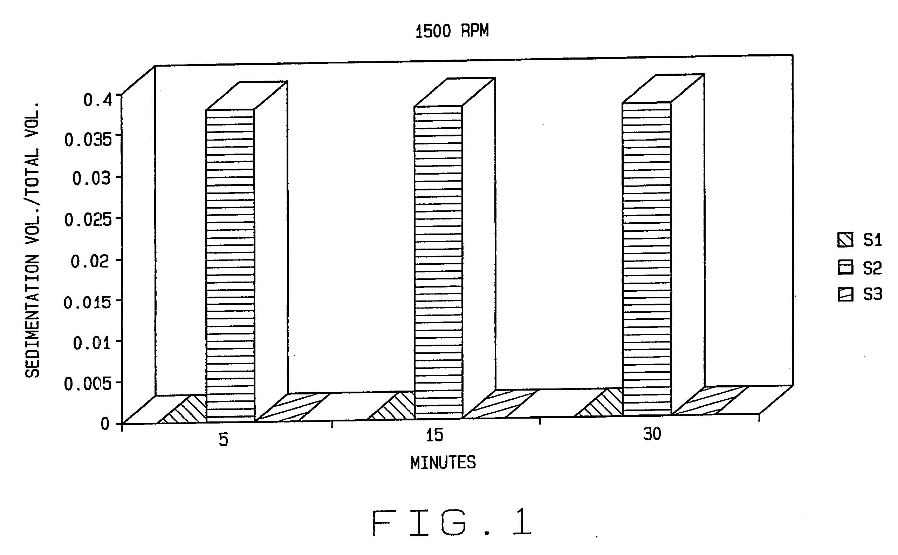

To assess the effects of colloidal silicon dioxide on sedimentation and physical stability, 10 ml aliquots of Celecoxib Suspensions S1-S3 were individually centrifuged at three different rates. At several time points during centrifugation, the resulting sediment volumes were quantified (Figs. 1-3). As shown in Fig. 1, after 5, 15 and 30 minutes of centrifugation at 1500 rpm, Celecoxib Suspension S2 (comprising colloidal silicon dioxide but no xanthan gum) resulted in a sediment volume of 0.0375 ml, whereas no sediment was observed in vessels containing Celecoxib Suspensions S 1 (xanthan gum but no colloidal silicon dioxide) and S3 (both xanthan gum and colloidal silicon dioxide).

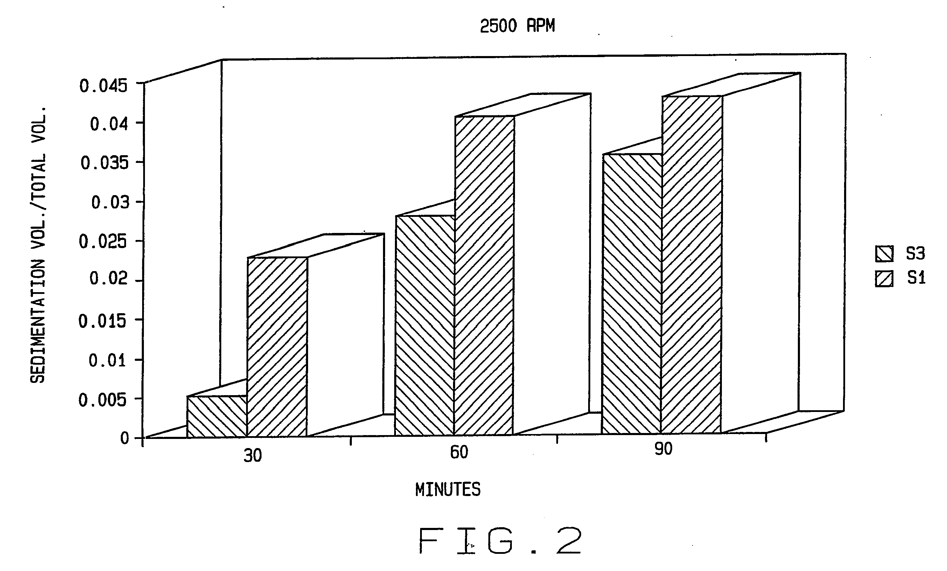

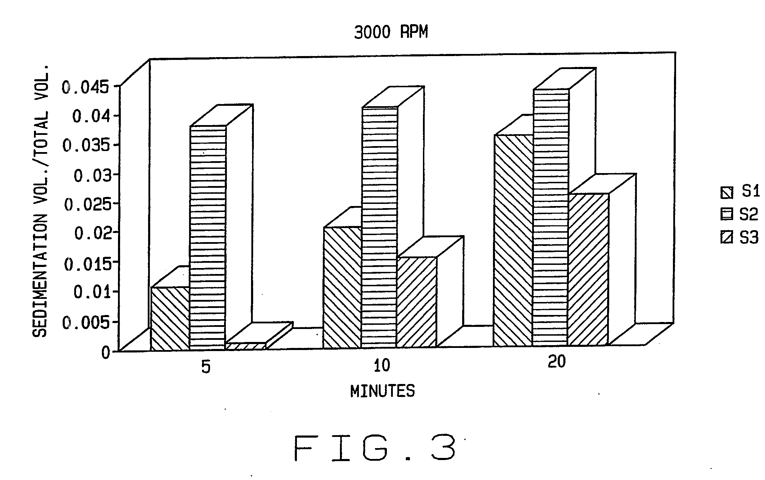

As shown in Fig. 2, after centrifugation at 2500 rpm for 30, 60 and 90 minutes, Celecoxib Suspension S1 exhibited greater sediment volume than did Celecoxib Suspension S3. Moreover, as shown in Fig. 3, centrifugation of Celecoxib Suspensions S 1 and S2 at 3,000 rpm for 5, 10 and 20 minutes resulted in greater sediment volume than did centrifugation of Celecoxib Suspension S3 under the same conditions.

These data indicate that presence of both xanthan gum and colloidal silicon dioxide greatly increase anti-sedimentation properties of Celecoxib Suspensions compared to suspensions comprising either xanthan gum or colloidal silicon dioxide alone. This anti-sedimentation effect indicates an increase in physical stability of the Celecoxib suspension.

### Example 4

Rheology of Celecoxib Suspensions S1-S4 and S6 was determined according to above-described Test I. Resulting dynamic viscosities, measured at 2 sec⁻¹, and yield values are shown in Table 3.

**Table 3. Rheology of Celecoxib Suspensions S1-S4 and S6.**

| **Celecoxib Suspension** | **Dynamic Viscosity (Pa•s) (at shear rate of 2 sec⁻¹)** | **Yield Value (Pa)** |
|---|---|---|
| **S1** | 3.21 | 5.3 |
| **S2** | 0.08 | 0.14 |
| **S3** | 3.58 | 6.2 |
| **S4** | 5.49 | 8.5 |
| **S6** | 0.13 | 0.16 |

As shown in Table 3, presence of both xanthan gum and colloidal silicon dioxide (Celecoxib Suspensions S3 and S4) had a synergistic effect on yield value as compared to the Celecoxib Suspension comprising colloidal silicon dioxide but no xanthan gum (S2 and S6) and the Celecoxib Suspension comprising xanthan gum but no colloidal silicon dioxide (S1). This increase in yield value indicates the presence of relatively high flow resistance at low stresses, for example gravitational stresses involved in particle sedimentation. Despite high yield values exhibited by Celecoxib Suspensions S3 and S4, dynamic viscosities, measured at a shear rate of 2 sec⁻¹, were still relatively low indicating good fluidity of the suspensions after yield value was reached. These data indicate that Celecoxib Suspensions S3 and S4 are thick at rest but fluid after moderate agitation. This increased thickness at rest will result in increased physical stability.

### Example 5

Five celecoxib suspensions, S7-S11, were prepared containing components shown in Table 4. All components were admixed, and suspensions were homogenized for 5 minutes using a Silverson Laboratory Mixer Emulsifier.

**Table 4. Composition (%) of Celecoxib Suspensions S7-S11.**

| **Component** | **S7** | **S8** | **S9*** | **S10** | **S11** |
|---|---|---|---|---|---|
| Celecoxib | 2 | 2 | 2 | 2 | 2 |
| Xanthan gum | 0.5 | 0.1 | 0.1 | 0.2 | 0.5 |
| Colloidal silica | 0.5 | 0.5 | - | 0.5 | 0.5 |
| Polysorbate 80 | 0.25 | 0.25 | 0.25 | 0.25 | 0.25 |
| Sucrose | 38.25 | 30 | 30 | 30 | 30 |
| Citric acid | 1.31 | 1.31 | 1.31 | 1.31 | 1.31 |
| Sodium citrate | 1.11 | 1.11 | 1.11 | 1.11 | 1.11 |
| Tutti fruti | 0.05 | 0.1 | 0.1 | 0.1 | 0.1 |
| Sodium benzoate | 0.2 | 0.2 | 0.2 | 0.2 | 0.2 |
| FD&C red #40 | 0.015 | 0.015 | 0.015 | 0.015 | 0.015 |
| 70% sorbitol solution | 45 | - | - | - | - |
| Water | to 100 | to 100 | to 100 | to 100 | to 100 |

| | | | | | |
|---|---|---|---|---|---|
| * For comparison. | | | | | |

### Example 6

Rheology of Celecoxib Suspensions S7-S11 was determined according to above-described Test I. Dynamic viscosities, measured at 2 sec⁻¹ , and yield values are shown in Table 5.

**Table 5. Rheology of Celecoxib Suspensions S7-S11.**

| **Celecoxib Suspension** | **Dynamic Viscosity (Pa•s) (at shear rate of 2 sec⁻¹)** | **Yield Value (Pa)** |
|---|---|---|
| **S7** | 22.04 | 19 |
| **S8** | 0.612 | 0.75 |
| **S9** | 0.184 | 0.19 |
| **S10** | 0.855 | 1.2 |
| **S11** | 3.566 | 5.9 |

As shown in Table 5, the presence of a very low amount of xanthan gum (0.2% or less) in Celecoxib Suspensions Sδ S9 and S 10 was insufficient, even with silicon dioxide present (S8 and S10), to impart suitable physical stability and thixotropic characteristics on the suspensions. In contrast, xanthan gum at 0.5% provided suitable rheologic characteristics for Celecoxib Suspension S11. Results for celecoxib Suspension S7 show suggest that high concentrations of sugar in compositions of the invention greatly increase both yield value and dynamic viscosity such that the suspension is not readily pourable even after mild agitation.

## Claims

1. A physically stable, thixotropic, orally deliverable pharmaceutical composition, wherein the composition comprises:
a drug having a solubility in water that is no greater than 10 mg/ml at 37°C ; and
an aqueous liquid vehicle that comprises:
(a) a pharmaceutically acceptable wetting agent,
(b) a pharmaceutically acceptable thixotropic thickening agent present in a thixotropic amount of from 0.4% to 2% by weight, and
(c) a pharmaceutically acceptable inorganic suspending agent; and
wherein the thixotropic thickening agent is xanthan gum and wherein the inorganic suspending agent is colloidal silicon dioxide.

2. A composition of Claim 1 wherein the drug is suspended in particulate form in the vehicle to form a suspension that is deflocculated.

3. A composition of Claim 1 wherein:
the drug is a selective cyclooxygenase-2 inhibitory drug that is a compound of formula: 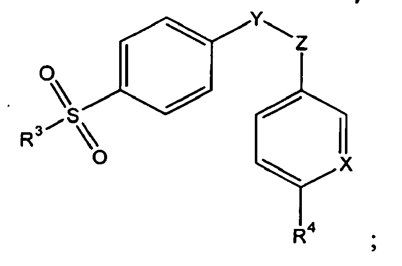
R³ is methyl or amino;
R⁴ is hydrogen, C₁₋₄ alkyl, or C₁₋₄ alkoxy;
X is N or CR⁵;
R⁵ is hydrogen or halogen; and
Y and Z are independently carbon or nitrogen atoms defining adjacent atoms of a five-to six-membered ring, wherein:
the five- to six-membered ring is unsubstituted or substituted with oxo, halo, methyl, or halomethyl, and
the five- to six-membered ring is selected from the group consisting of cyclopentenone, furanone, methylpyrazole, isoxazole, and pyridine.

4. A composition of Claim 1 wherein the drug comprises a selective cyclooxygenase-2 inhibitory drug selected from the group consisting of celecoxib, deracoxib, valdecoxib, rofecoxib, etoricoxib, 2-(3,5-difluorophenyl)-3-[4-(methylsulfonyl)phenyl]-2-cyclopenten-1-one, and (S)-6,8-dichloro-2-(trifluoromethyl)-2H-1-benzopyran-3-carboxylic acid.

5. A composition of Claim 1 wherein the wetting agent is selected from the group consisting of quaternary ammonium compounds, dioctyl sodium sulfosuccinate, polyoxyethylene alkylphenyl ethers, poloxamers, polyoxyethylene fatty acid glycerides and oils, polyoxyethylene alkyl ethers, polyoxyethylene fatty acid esters, polyoxyethylene sorbitan esters, propylene glycol fatty acid esters, sodium lauryl sulfate, fatty acids and salts thereof, glyceryl fatty acid esters, sorbitan esters, tyloxapol and mixtures thereof.

6. A composition of Claim 1 wherein:
the drug is present in an amount of 0.01% to 15%, by weight,
the wetting agent is present in an amount of 0.01% to 2%, by weight, and
the inorganic suspending agent is present in an amount of 0.01% to 3%, by weight.

7. A method for increasing the physical stability of an aqueous, thixotropic pharmaceutical composition, wherein:
the composition comprises a drug having a solubility in water that is no greater than 10 mg/ml at 37°C,
the method comprises adding to the composition:
a pharmaceutically acceptable thixotropic thickening agent in a thixotropic amount of 0.4% to 2% (by weight) and a pharmaceutically acceptable inorganic suspending agent in an amount of from 0.01 % to 3.0% such that the thixotropic thickening agent and inorganic suspending agent act synergistically to increase physical stability of the composition, and
a wetting agent; and
wherein the thixotropic thickening agent is xanthan gum and wherein the inorganic suspending agent is colloidal silicon dioxide.

8. A method of Claim 7 wherein the drug is suspended in particulate form in the mixture to form a suspension that is deflocculated.

9. A method of Claim 7 wherein:
the drug is a selective cyclooxygenase-2 inhibitory drug that is a compound of formula: 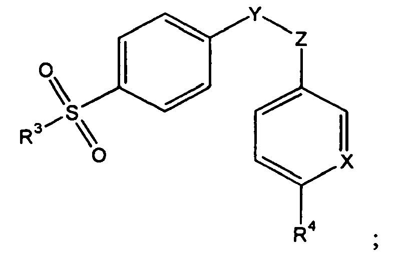
R³ is methyl or amino;
R⁴ is hydrogen, C₁₋₄ alkyl, or C₁₋₄ alkoxy;
X is N or CR⁵;
R⁵ is hydrogen or halogen; and
Y and Z are independently carbon or nitrogen atoms defining adjacent atoms of a five-to six-membered ring, wherein:
the five- to six-membered ring is unsubstituted or substituted with oxo, halo, methyl, or halomethyl, and
the five- to six-membered ring is selected from the group consisting of cyclopentene, dihydrofuran, pyrazole, isoxazole, and pyridine.

10. A method of Claim 7 wherein the drug comprises a selective cyclooxygenase-2 inhibitory drug selected from the group consisting of celecoxib, deracoxib, valdecoxib, rofecoxib, etoricoxib, 2-(3,5-difluorophenyl)-3-[4-(methylsulfonyl)phenyl]-2-cyclopenten-1-one, and (S)-6,8-dichloro-2-(trifluoromethyl)-2H-1-benzopyran-3-carboxylic acid.

11. A method of Claim 7 wherein the wetting agent is selected from the group consisting of quaternary ammonium compounds, dioctyl sodium sulfosuccinate, polyoxyethylene alkylphenyl ethers, poloxamers, polyoxyethylene fatty acid glycerides and oils, polyoxyethylene alkyl ethers, polyoxyethylene fatty acid esters, polyoxyethylene sorbitan esters, propylene glycol fatty acid esters, sodium lauryl sulfate, fatty acids and salts thereof, glyceryl fatty acid esters, sorbitan esters, tyloxapol and mixtures thereof.

12. A method of Claim 7 wherein:
the drug is present in an amount of 0.01 % to 15%, by weight, and
the wetting agent is present in an amount of 0.01% to 2%, by weight.

13. A physically stable, thixotropic, orally deliverable pharmaceutical composition of Claim 1 wherein:
the pharmaceutically acceptable thixotropic thickening agent is present in thixotropic amount of from 0.25% to 2% by weight, and
the drug comprises a compound of formula: 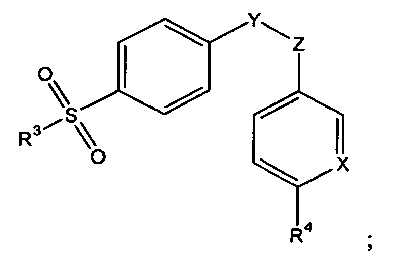
R³ is methyl or amino;
R⁴ is hydrogen, C₁₋₄ alkyl, or C₁₋₄ alkoxy;
X is N or CR⁵;
R⁵ is hydrogen or halogen; and
Y and Z are independently carbon or nitrogen atoms defining adjacent atoms of a five-to six-membered ring, wherein:
the five- to six-membered ring is unsubstituted or substituted with oxo, halo, methyl, or halomethyl.

14. A method of Claim 7 wherein:
the method comprises adding to the composition:
the pharmaceutically acceptable thixotropic thickening agent in a thixotropic amount of from 0.25% to 2% (by weight); and
the drug comprises a compound of formula: 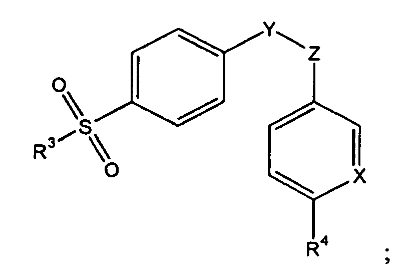
R³ is methyl or amino;
R⁴ is hydrogen, C₁₋₄ alkyl, or C₁₋₄ alkoxy;
X is N or CR⁵;
R⁵ is hydrogen or halogen; and
Y and Z are independently carbon or nitrogen atoms defining adjacent atoms of a five-to six-membered ring, wherein:
the five- to six-membered ring is unsubstituted or substituted with oxo, halo, methyl, or halomethyl.

## Patentansprüche

1. Physikalisch stabile, thixotrope, oral zuführbare pharmazeutische Zusammensetzung, wobei die Zusammensetzung umfasst:
Einen Wirkstoff, der eine Löslichkeit in Wasser hat, die nicht größer als 10 mg/ml bei 37°C ist, und ein wässriges flüssiges Vehikel, das umfasst:
(a) ein pharmazeutisch annehmbares Netzmittel,
(b) ein pharmazeutisch akzeptables thixotropes Verdickungsmittel, das in einer thixotropen Menge von 0,4 Gewichts-% bis 2 Gewichts-% vorliegt, und
(c) ein pharmazeutisch akzeptables anorganisches Suspendiermittel;
und wobei das thixotrope Verdickungsmittel Xanthangummi ist und wobei das anorganische Suspendiermittel kolloidales Siliciumdioxid ist.

2. Zusammensetzung nach Anspruch 1, wobei der Wirkstoff in partikelförmiger Form unter Bildung einer Suspension, die entflockt ist, in dem Vehikel suspendiert ist.

3. Zusammensetzung nach Anspruch 1, wobei:
der Wirkstoff ein selektiver Cyclooxygenase-2-Inhibitorwirkstoff ist, der eine Verbindung der Formel: 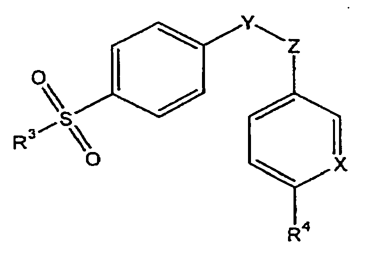
ist;
wobei R³ Methyl oder Amino ist;
R⁴ Wasserstoff, C₁₋₄-Alkyl oder C₁₋₄-Alkoxy ist;
X N oder CR⁵ ist;
R⁵ Wasserstoff oder Halogen ist und
Y und Z unabhängig Kohlenstoff- oder Stickstoffatome sind, die benachbarte Atome eines fünf- bis sechs-gliedrigen Rings definieren, wobei:
der fünf- bis sechs-gliedrige Ring unsubstituiert ist oder mit Oxo, Halogen, Methyl oder Halogenmethyl substituiert ist und
der fünf- bis sechs-gliedrige Ring ausgewählt ist aus der Gruppe, bestehend aus Cyclopentenon, Furanon, Methylpyrazol, Isoxazol und Pyridin.

4. Zusammensetzung nach Anspruch 1, wobei der Wirkstoff einen selektiven Cyclooxygenase-2-Inhibitorwirkstoff umfasst, der ausgewählt ist aus der Gruppe, bestehend aus Celecoxib, Deracoxib, Valdecoxib, Rofecoxib, Etoricoxib, 2-(3,5-Difluorphenyl)-3-[4-(methylsulfonyl)phenyl]-2-cyclopenten-1-on und (S)-6,8-Dichlor-2-(trifluormethyl)-2H-1-benzopyran-3-carbonsäure.

5. Zusammensetzung nach Anspruch 1, wobei das Netzmittel ausgewählt ist aus der Gruppe, bestehend aus quaternären Ammoniumverbindungen, Dioctylnatriumsulfosuccinat, Polyoxyethylenalkylphenylethern, Poloxameren, Polyoxyethylenfettsäureglyceriden und -ölen, Polyoxyethylenalkylethern, Polyoxyethylenfettsäureestern, Polyoxyethylensorbitanestern, Propylenglycolfettsäureestern, Natriumlaurylsulfat, Fettsäuren und Salzen davon, Glycerylfettsäureestern, Sorbitanestern, Tyloxapol und Gemischen davon, ausgewählt ist.

6. Zusammensetzung nach Anspruch 1, wobei:
der Wirkstoff in einer Menge von 0,01 Gewichts-% bis 15 Gewichts-% vorhanden ist,
das Netzmittel in einer Menge von 0,01 Gewichts-% bis 2 Gewichts-% vorhanden ist und
das anorganische Suspendiermittel in einer Menge von 0,01 Gewichts-% bis 3 Gewichts-% vorhanden ist.

7. Verfahren zur Erhöhung der physikalischen Stabilität einer wässrigen, thixotropen pharmazeutischen Zusammensetzung, wobei:
die Zusammensetzung einen Wirkstoff umfasst, der eine Löslichkeit in Wasser hat, die nicht größer als 10 mg/ml bei 37°C ist,
wobei das Verfahren Zusetzen zu der Zusammensetzung: eines pharmazeutisch akzeptablen thixotropen Verdickungsmittels in einer thixotropen Menge von 0,4% bis 2% (auf das Gewicht bezogen) und eines pharmazeutisch akzeptablen anorganischen Suspendiermittels in einer Menge von 0,01% bis 3,0%, so dass das thixotrope Verdickungsmittel und das anorganische Suspendiermittel synergistisch unter Erhöhung der physikalischen Stabilität der Zusammensetzung wirken, und
eines Netzmittels umfasst; und
wobei das thixotrope Verdickungsmittel ein Xanthangummi ist und wobei das anorganische Suspendiermittel kolloidales Siliciumdioxid ist.

8. Verfahren nach Anspruch 7, wobei der Wirkstoff in partikelförmiger Form in dem Gemisch unter Bildung einer Suspension, die entflockt ist, suspendiert wird.

9. Verfahren nach Anspruch 7, wobei:
der Wirkstoff ein selektiver Cyclooxygenase-2-Inhibitorwirkstoff ist, der eine Verbindung der Formel: 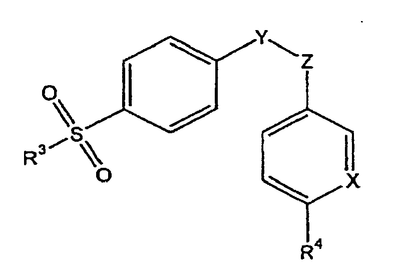
ist, wobei
R³ Methyl oder Amino ist;
R⁴ Wasserstoff, C₁-₄-Alkyl oder C₁₋₄-Alkoxy ist;
X N oder CR⁵ ist;
R⁵ Wasserstoff oder Halogen ist und
Y und Z unabhängig Kohlenstoff- oder Stickstoffatome sind, die benachbarte Atome eines fünf- bis sechs-gliedrigen Rings definieren, wobei:
der fünf- bis sechs-gliedrige Ring unsubstituiert ist oder mit Oxo, Halogen, Methyl oder Halogenmethyl substituiert ist und
der fünf- bis sechs-gliedrige Ring aus der Gruppe, bestehend aus Cyclopenten, Dihydrofuran, Pyrazol, Isoxazol und Pyridin, ausgewählt ist.

10. Verfahren nach Anspruch 7, wobei der Wirkstoff einen selektiven Cyclooxygenase-2-Inhibitorwirkstoff umfasst, der aus der Gruppe, bestehend aus Celecoxib, Deracoxib, Valdecoxib, Rofecoxib, Etoricoxib, 2-(3,5-Difluorphenyl)-3-[4-(methylsulfonyl)phenyl]-2-cyclopenten-1-on und (S)-6,8-Dichlor-2-(trifluormethyl)-2H-1-benzopyran-3-carbonsäure, ausgewählt wird.

11. Verfahren nach Anspruch 7, wobei das Netzmittel ausgewählt ist aus der Gruppe, bestehend aus quaternären Ammoniumverbindungen, Dioctylnatriumsulfosuccinat, Polyoxyethylenalkylphenylethern, Poloxameren, Polyoxyethylenfettsäureglyceriden und -ölen, Polyoxyethylenalkylethern, Polyoxyethylenfettsäureestern, Polyoxyethylensorbitanestern, Propylenglycolfettsäureestern, Natriumlaurylsulfat, Fettsäuren und Salzen davon, Glyerylfettsäureestern, Sorbitanestern, Tyloxapol und Gemischen davon.

12. Verfahren nach Anspruch 7, wobei:
der Wirkstoff in einer Menge von 0,01 Gewichts-% bis 15 Gewichts-% vorliegt und
das Netzmittel in einer Menge von 0,01 Gewichts-% bis 2 Gewichts-% vorliegt.

13. Physikalisch stabile, thixotrope, oral zuführbare pharmazeutische Zusammensetzung nach Anspruch 1, wobei:
das pharmazeutisch akzeptable thixotrope Verdickungsmittel in einer thixotropen Menge von 0,25 Gewichts-% bis 2 Gewichts-% vorhanden ist und
der Wirkstoff eine Verbindung der Formel umfasst: 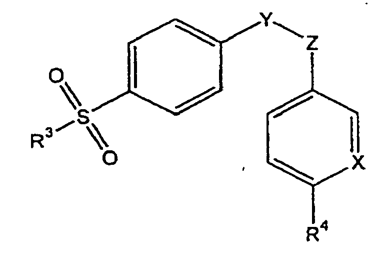
wobei:
R³ Methyl oder Amino ist;
R⁴ Wasserstoff, C₁₋₄-Alkyl oder C₁₋₄-Alkoxy ist;
X N oder CR⁵ ist;
R⁵ Wasserstoff oder Halogen ist und
Y und Z unabhängig Kohlenstoff- oder Stickstoffatome sind, die benachbarte Atome eines fünf- oder sechs-gliedrigen Rings definieren, wobei:
der fünf- bis sechs-gliedrige Ring unsubstituiert ist oder mit Oxo, Halogen, Methyl oder Halogenmethyl substituiert ist.

14. Verfahren nach Anspruch 7, wobei das Verfahren Zusetzen zu der Zusammensetzung:
des pharmazeutisch akzeptablen thixotropen Verdickungsmittels in einer thixotropen Menge von 0,25% bis 2% (bezogen auf das Gewicht) umfasst und
der Wirkstoff eine Verbindung der Formel umfasst: 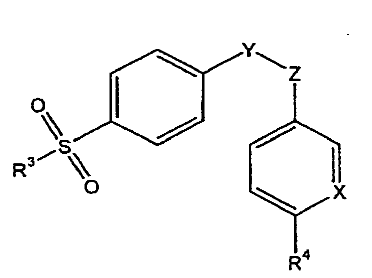
wobei:
R³ Methyl oder Amino ist;
R⁴ Wasserstoff, C₁₋₄-Alkyl oder C₁₋₄-Alkoxy ist;
X N oder CR⁵ ist;
R⁵ Wasserstoff oder Halogen ist und
Y und Z unabhängig Kohlenstoff- oder Stickstoffatome sind, die benachbarte Atome eines fünf- bis sechs-gliedrigen Rings definieren, wobei:
der fünf- bis sechs-gliedrige Ring unsubstituiert ist oder mit Oxo, Halogen, Methyl oder Halogenmethyl substituiert ist.

## Revendications

1. Composition pharmaceutique physiquement stable, thixotrope, destinée à être administrée par voie orale, dans laquelle la composition comprend :
un médicament présentant une solubilité dans l'eau qui est inférieure à 10 mg/ml à 37° C ; et un véhicule liquide aqueux qui comprend :
(a) un agent mouillant pharmaceutiquement acceptable,
(b) un agent épaississant thixotrope pharmaceutiquement acceptable présent en une quantité thixotrope de 0,4 % à 2 % en poids, et
(c) un agent de suspension inorganique pharmaceutiquement acceptable ; et dans lequel l'agent épaississant thixotrope est la gomme xanthane et dans lequel l'agent de suspension inorganique est un dioxyde de silicium colloïdal.

2. Composition selon la revendication 1, dans laquelle le médicament est mis en suspension sous forme particulaire dans un véhicule pour former une suspension qui est défloculée.

3. Composition selon la revendication 1, dans laquelle
le médicament est un inhibiteur sélectif de la cyclo-oxygénase-2 qui est un composé de formule 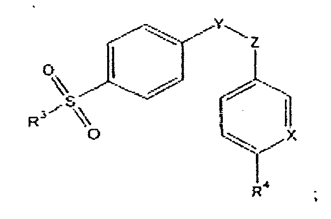 dans laquelle
R³ est un groupe méthyle ou amino ;
R⁴ est un atome d'hydrogène, un groupe alkyle en C₁ à C₄ ou alcoxy en C₁ à C₄ ;
X est N ou CR⁵;
R⁵ est un atome d'hydrogène ou d'halogène ; et
Y et Z sont indépendamment les atomes de carbone ou d'azote définissant des atomes adjacents d'un cycle comportant cinq à six éléments, dans lesquels
le cycle comportant cinq à six éléments est non substitué ou substitué par des groupes oxo, halo, méthyle, ou halométhyle, et
le cycle comportant cinq à six éléments est choisi dans le groupe constitué du cyclopenténone, du furanone, du méthylpyrazole, de l'isoxazole et de la pyridine.

4. Composition selon la revendication 1, dans laquelle le médicament comprend un inhibiteur sélectif de la cyclo-oxygénase-2 choisi dans le groupe constitué du célécoxib, du deracoxib, du valdécoxib, du rofécoxib, de l'étoricoxib, du 2-(3,5-difluorophényl)-3-[4-(méthylsulfonyl)phényl]-2-cyclopentén-1-one et de l'acide (S)-6,8-dichloro-2-(trifluorométhyl)-2H-1-benzopyrane-3-carboxylique.

5. Composition selon la revendication 1, dans laquelle l'agent mouillant est choisi dans le groupe constitué des composés d'ammonium quaternaire, du sulfosuccinate de dioctyl-sodium, des éthers de polyoxy-éthylène-alkylphényle, des poloxamères, des glycérides d'acide gras et huiles polyoxyéthylène, des éthers de polyoxy-éthylène-alkyle, des esters d'acides gras polyoxy-éthylène, des esters de polyoxy-éthylène sorbitan, des esters d'acides gras de propylène glycol, du lauryl-sulfate de sodium, de leurs acides gras et sels, des esters d'acides gras de glycéryle, des esters de sorbitan, du tyloxapol et de leurs mélanges.

6. Composition selon la revendication 1, dans laquelle
le médicament est présent en une quantité de 0,01 % à 15 % en poids,
l'agent mouillant est présent en une quantité de 0,01 % à 2 % en poids, et
l'agent de suspension inorganique est présent en une quantité de 0,01 % à 3 % en poids.

7. Procédé d'augmentation de la stabilité physique d'une composition pharmaceutique aqueuse, thixotrope, dans laquelle
la composition comprend un médicament présentant une solubilité dans l'eau qui est inférieure à 10 mg/ml à 37° C,
le procédé comprenant l'addition à la composition, d'un agent épaississant thixotrope pharmaceutiquement acceptable en une quantité thixotrope de 0,4 % à 2 % (en poids) et un agent de suspension inorganique pharmaceutiquement acceptable en une quantité de 0,01 % à 3,0 % tel que l'agent épaississant thixotrope et l'agent de suspension inorganique agissent en synergie pour augmenter la stabilité physique de la composition, et un agent mouillant ; et dans laquelle l'agent épaississant thixotrope est la gomme xanthane et dans laquelle l'agent de suspension inorganique est le dioxyde de silicium colloïdal.

8. Procédé selon la revendication 7, dans lequel le médicament est mis en suspension sous forme particulaire dans le mélange pour former une suspension qui est défloculée.

9. Procédé selon la revendication 7, dans lequel
le médicament est un inhibiteur sélectif de la cyclo-oxygénase-2 qui est un composé de formule : 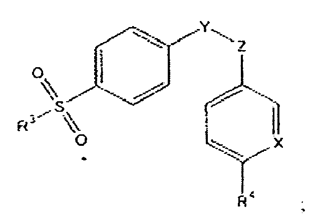
R³ est un groupe méthyle ou amino ;
R⁴ est un atome d'hydrogène, un groupe alkyle en C₁ à C₄ ou alcoxy en C₁ à C₄ ;
X est N ou CR⁵ ;
R⁵ est un atome d'hydrogène ou d'halogène ; et
Y et Z sont indépendamment les atomes de carbone ou d'azote définissant des atomes adjacents d'un cycle comportant cinq à six éléments, dans lesquels
le cycle comportant cinq à six éléments est non substitué ou substitué par des groupes oxo, halo, méthyle, ou halométhyle, et
le cycle comportant cinq à six éléments est choisi dans le groupe constitué du cyclopenténone, du furanone, du méthylpyrazole, de l'isoxazole et de la pyridine.

10. Procédé selon la revendication 7, dans lequel le médicament comprend un inhibiteur sélectif de la cyclo-oxygénase-2 choisi dans le groupe constitué du célécoxib, du deracoxib, du valdécoxib, du rofécoxib, de l'étoricoxib, du 2-(3,5-difluorophényl)-3-[4-(méthylsulfonyl)phényl]-2-cyclopentén-1-one et de l'acide (S)-6,8-dichloro-2-(trifluorométhyl)-2H-1-benzopyrane-3-carboxylique.

11. Procédé selon la revendication 7, dans lequel l'agent mouillant est choisi dans le groupe constitué des composés d'ammonium quaternaire, du sulfosuccinate de dioctyl-sodium, des éthers de polyoxy-éthylène-alkylphényle, des poloxamères, des glycérides d'acide gras et huiles polyoxyéthylène, des éthers de polyoxy-éthylène-alkyle, des esters d'acides gras polyoxy-éthylène, des esters de polyoxy-éthylène sorbitan, des esters d'acides gras de propylène glycol, du lauryl-sulfate de sodium, de leurs acides gras et sels, des esters d'acides gras de glycéryle, des esters de sorbitan, du tyloxapol et de leurs mélanges.

12. Procédé selon la revendication 7, dans lequel le médicament est présent en une quantité de 0,01 % à 15 % en poids, et
l'agent mouillant est présent en une quantité de 0,01 % à 2 % en poids.

13. Composition pharmaceutique physiquement stable, thixotrope destinée à l'administration orale, selon la revendication 1 dans laquelle
l'agent épaississant thixotrope pharmaceutiquement acceptable est présent en quantité thixotrope de 0,25 % à 2 % en poids, et
le médicament comprend un composé de formule : 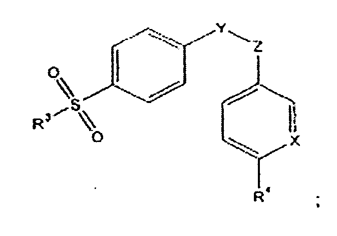 dans laquelle
R³ est un groupe méthyle ou amino ;
R⁴ est un atome d'hydrogène, un groupe alkyle en C₁ à C₄ ou alcoxy en C₁ à C₄ ;
X est N ou CR⁵ ;
R⁵ est un atome d'hydrogène ou d'halogène ; et
Y et Z sont indépendamment les atomes de carbone ou d'azote définissant des atomes adjacents d'un cycle comportant cinq à six éléments, dans lesquels
le cycle comportant cinq à six éléments est non substitué ou substitué par des groupes oxo, halo, méthyle, ou halométhyle.

14. Procédé selon la revendication 7, dans lequel
le procédé comprend l'addition à la composition de l'agent épaississant thixotrope pharmaceutiquement acceptable en une quantité thixotrope de 0,25 % à 2 % (en poids) ; et
le médicament comprend un composé de formule : 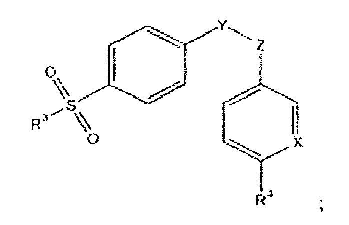 dans laquelle
R³ est un groupe méthyle ou amino ;
R⁴ est un atome d'hydrogène, un groupe alkyle en C₁ à C₄ ou alcoxy en C₁ à C₄ ;
X est N ou CR⁵ ;
R⁵est un atome d'hydrogène ou d'halogène ; et
Y et Z sont indépendamment les atomes de carbone ou d'azote définissant des atomes adjacents d'un cycle comportant cinq à six éléments, dans lesquels
le cycle comportant cinq à six éléments est non substitué ou substitué par des groupes oxo, halo, méthyle, ou halométhyle.
